# EUROPEAN PATENT SPECIFICATION

(11) **EP 1 996 244 B1**
(45) Date of publication and mention of the grant of the patent: **25.01.2012**
(21) Application number: 07752379.3
(22) Date of filing: 05.03.2007
(51) Int. Cl.: A61L 27/34, A61L 27/58, A61L 31/10

(54) **CORROSION RESISTANT COATINGS COMPRISING ELECTRICALLY CONDUCTIVE POLYMER FOR BIODEGRADABLE METALLIC STENTS**
KORROSIONSRESISTENTE BESCHICHTUNG MIT ELEKTRISCH LEITENDEM POLYMER FÜR BIOLOGISCH ABBAUBARE METALLISCHE STENTS
REVETEMENTS RESISTANTs A LA CORROSION COMPRENANT UN POLYMERE CONDUCTEUR ELECTRIQUE POUR DES STENTS METALLIQUES BIODEGRADABLES

(30) Priority: 22.03.2006 US 387032
(43) Date of publication of application: 03.12.2008
(73) Proprietor: Boston Scientific Limited, Christ Church (BB)
(72) Inventor: WEBER, Jan, NL-6228 GJ Maastricht (NL); ATANASOSKA, Liliana, Edina, MN 55436 (US); EIDENSCHINK, Tracee, Wayzata, MN 55391 (US)
(74) Representative: Peterreins, Frank
(86) International application number: PCT/US2007/005671
(87) International publication number: WO 2007/111811

(56) References cited:
- WO-A-03/035278
- US-A1- 2002 004 060
- US-A1- 2003 204 239
- US-A1- 2005 209 680
- Z. WEISS, D. MANDLER, G. SHUSTAK, A.J. DOMB: "Pyrrole derivatives for electrochemical coating of metallic medical devices" JOURNAL OF POLYMER SCIENCE, PART A, vol. 42, 17 November 2003 (2003-11-17), pages 1658-1667, XP002480804
- T.R. FARHAT, J.B. SCHLENOFF: "Corrosion control using polyelectrolyte multilayers" ELECTROCHEMICAL AND SOLID-STATE LETTERS, vol. 5, no. 4, 4 February 2002 (2002-02-04), pages B13-B15, XP002480805

## Description

### FIELD OF THE INVENTION

The present invention relates to implantable stents and more particularly to biodegradable metallic implantable stents.

### BACKGROUND

One of the latest developments in metallic implant designs is based on metallic alloys that degrade in vivo. For example as described in U.S. Patent App. Pub. No. 2002/0004060 AI, entitled "Metallic implant which is degradable in vivo" implants may be formed from pure metals or metal alloys whose main constituent is selected from alkali metals, alkaline earth metals, iron, and zinc. Particularly preferred are metals and metal alloys containing magnesium, iron or zinc as a main constituent and one or more additional constituents selected from the following: alkali metals such as Li, alkaline-earth metals such as Ca and Mg, transition metals such as Mn, Co, Ni, Cr, Cu, Cd, Zr, Ag, Au, Pd, pt, Re, Fe and Zn, Group IIIa metals such as AI, and Group IVa elements such as C, Si, Sn and Pb. Of these, magnesium alloys have high corrosion rates, particularly in the presence of NaCI, which is found in vivo.
US 2005/0209680 A1 discloses a device and a method of manufacturing an implantable medical device, such as a stent. The device includes a metallic region composed of a bioerodable metal and a polymer region composed of a biodegradable polymer contacting the metallic region. The metallic region may erode at a different rate when exposed to bodily fluids than the polymer region when exposed to bodily fluids. In certain embodiments, the polymer region is an outer layer and the metallic region is an inner layer of the device. A further aspect of the invention includes a device and a method of manufacturing the device that includes a mixture of a biodegradable polymer and bioerodable metallic particles. The mixture may be used to fabricate an implantable medical device or to coat an implantable medical device.

### SUMMARY OF THE INVENTION

The invention relates to an implantable stent as described in claim 1.

According to an aspect of the invention, implantable stents are provided which contain (a) at least one biodegradable metallic region and (b) a polymeric corrosion resistant coating over the biodegradable metallic region. The polymeric corrosion resistant coating slows the rate of corrosion of the biodegradable metallic region after implantation into a subject.

An advantage of the invention is that corrosion rates may be controlled in metallic regions of implantable stents, without the need to alter the bulk properties of the metallic regions.

Another advantage of the present invention is that corrosion may be controlled such that it preferentially occurs at predefined locations on the implants, if desired.

These and other aspects, embodiments and advantages of the present invention will become immediately apparent to those of ordinary skill in the art upon reading the disclosure to follow.

### BRIEF DESCRIPTION OF THE DRAWINGS

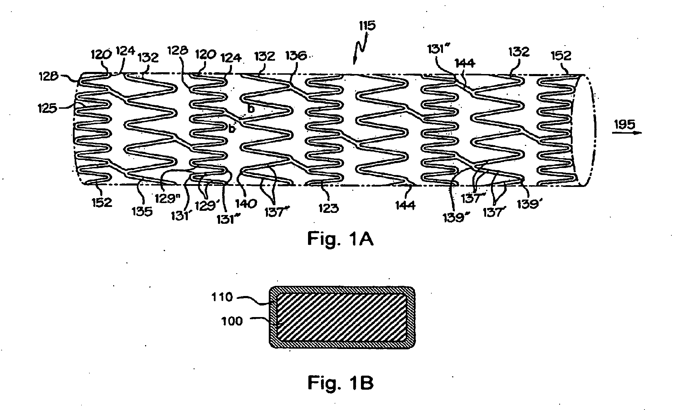

Fig. 1A is a schematic view ofa stent, in accordance with an embodiment of the invention.

Fig. 1B is a cross-sectional view taken along line b-b of Fig. 1A.

### DETAILED DESCRIPTION OF THE INVENTION

According to an aspect of the invention, implantable stents ("implants") are provided which contain at least one biodegradable metallic region and an electrically conductive polymeric corrosion resistant coating (also referred to herein as "coatings," "polymeric coatings," "protective coatings," "corrosion protective coatings" and the like) over the biodegradable metallic region. The corrosion resistant coating slows the rate of corrosion of the biodegradable metallic region upon implantation into a subject. Preferred subjects into whom the implants of the present invention may be introduced are vertebrate subjects, more preferably mammalian subjects, and even more preferably human subjects.

At first blush, the use of corrosion resistant coatings for biodegradable metallic regions of implants seems incongruous, because it appears to be antithetical to the goal of having the metallic regions biodegrade (i.e., corrode) in vivo. However, for partially or completely biodegradable implants (e.g., vascular stents, etc.), it is actually very useful in some instances to delay corrosion for a certain timeframe (e.g., until vessel patency is restored, etc.), but not indefinitely, which is the intent of conventional corrosion resistant coatings.

As used herein a "metallic" material is one that contains one or more metals, and commonly contains at 50 wt% to 75 wt%, to 90 wt% to 95 wt% to 99 wt% or even more, metals. Hence metallic materials include pure metals and metal alloys of two or more metals. Moreover, metallic materials may contain various optional non-metal additives.

As used herein a metallic "region" can correspond, for instance, to an entire implant (other than the corrosion resistant coating). On the other hand, a metallic region can correspond, for instance, to only a portion of an implant. For example, a metallic region can correspond to a metallic component of an implant, or it can be in the form of a metallic layer that is provided over an underlying substrate, for example, to give temporary rigidity to the underlying substrate. As used herein a "layer" of a given material is a region of that material whose thickness is small compared to both its length and width. As used herein a layer need not be planar, for example, taking on the contours of an underlying substrate.

Specific biodegradable metallic materials for use in the present invention include those set forth in U.S. Patent App. Pub. No. 2002/0004060 A1, and include pure metals and metal alloys having a main constituent (e.g., 50 wt% or more, in some cases) selected from alkali metals, alkaline earth metals, iron, and zinc. Examples include metals and metal alloys containing (a) magnesium, as a main constituent and (b) one or more additional constituents selected from the following: alkali metals such as Li, alkaline-earth metals such as Ca and Mg, transition metals such as Mn, Co, Ni, Cr, Cu, Cd, Zr, Ag, Au, Pd, Pt, Re, Fe and Zn, Group IIIa metals such as A1, Group IVa elements (e.g., metals and semiconductors) such as C, Si, Sn and Pb, among others. Specific examples of biodegradable metallic materials include the following, among others: material containing 50-98% magnesium, 0-40% lithium, 0-5% iron and less than 5% other metals or rare earths, material containing 79-97% magnesium, 2-5% aluminum, 0-12% lithium and 1-4% rare earths, in particular cerium, lanthanum, neodymium and/or praseodymium, material containing 85-91 % magnesium, 6-12% lithium, 2% aluminum and 1% rare earths, material containing 86-97% magnesium, 0-8% lithium, 2% -4% aluminum and 1-2% rare earths, material containing 55-65% magnesium, 30-40% lithium and 0-5% other metals and/or rare earths.

Specific materials for the present invention further include magnesium metal and magnesium metal alloys that contain one or more of the following: Ce, Ca, Zn, Fe, Zr, Mn, Al, and Li, among others.

Biodegradable metallic materials may contain other non-metallic species. Examples include alloys with non-metallic constituents such as carbon (see above, for example), among other elements. Further examples include composite materials having distinct biodegradable metallic regions and distinct non-metallic regions, which may be, for example, in the form of particles, fibers, tubes, and so forth. See, e.g., Yan Feng et al., "Superplasticity and texture of SiC whiskers in a magnesium-based composite," Scripta Materialia 53 (2005) 361-365; Q.C. Jiang et al., "Effect of TiB2 particulate on partial remelting behavior of Mg-11A1-0.5Zn matrix composite," Materials Science and Engineering A 381 (2004) 223-229; M. Russell-Stevens et al., "The effect of thermal cycling on the properties of a carbon fibre reinforced magnesium composite," Materials Science and Engineering A 397 (2005) 249-256; H.Z Ye et al., "In situ synthesis of AIN particles in Mg-Al alloy by Mg3N2 addition, " Materials Letters 58 (2004) 2361- 2364.

Implants for the present invention are stents (including coronary vascular stents, cerebral, urethral, ureteral, biliary, tracheal, gastrointestinal and esophageal stents).

An embodiment of the present invention will now be described in conjunction with a stent structure like that shown in Figs. 1A and 1B. The stent of Fig. 1 comprises cylindrical shaped first segments 120 which are defined by an undulating pattern of interconnected paired first struts 123 in which adjacent pairs of first struts 129' and 129" in a given first segment 120 are interconnected at opposite ends 131' and 131", respectively. The undulations are characterized by a plurality of peaks 124 and troughs 128 taking a generally longitudinal direction along the cylinder surface such that the waves in first segments 120 open as the stent is expanded from an unexpanded state having a first profile to an expanded state having a second profile. The stent further comprises one or more cylindrical shaped second segments 132, each second segment being defined by a member formed in an undulating pattern of interconnected paired second struts 135 and in which adjacent pairs of second struts 137' and 137" in a given second segment 132 are interconnected at opposite ends 139' and 139", respectively. The undulations in the second segments are characterized by a plurality of peaks 136 and troughs 140 taking a generally longitudinal direction along the cylinder such that the waves in the second segments 132 open as the stent is expanded from an unexpanded state having a first diameter to an expanded state having a second diameter. First segments 120 are formed of a number of first struts 123 and second segments 132 formed of a number of second struts 135. First struts 123 are shorter than second struts 135. First and second segments 120 and 132 are aligned on a common longitudinal axis 195 to define a generally tubular stent body, shown generally at 115, having ends 152. First and second segments 120 and 132 alternate along the stent body. Adjacent first and second segments 120 and 132 are connected by a plurality of interconnecting elements 144. Each interconnecting element 144 extends from an end 131" of paired first struts on a first segment 120 to an end 139" of paired second struts on an adjacent second segment 132. The ends of interconnecting elements 144 arc circumferentially offset relative to each other.

The overall structure of the stent of Figs. 1A and 1B is analogous to that described in U.S. Patent Pub. No. 2004/0181276. However, as seen from the cross-sectional view of Fig. 1B, the stent's structural elements comprise a biodegradable metallic region 100, which may be formed, for example, from the biodegradable metallic materials described above. Over the biodegradable metallic region 100 is provided a corrosion resistant coating 110. Various embodiments for polymeric corrosion resistant coatings are described in detail below.

As used herein a "polymeric" material is one that contains one or more types of polymers, commonly containing at 50 wt% to 75 wt%, to 90 wt% to 95 wt% to 99 wt% or even more polymers. Thus polymeric materials include those containing single type of polymer as well as polymer blends. Moreover polymeric materials may contain various optional non-polymer components, as described below.

As used herein, "polymers" are molecules that contain multiple copies of one or more constitutional units, commonly referred to as monomers, and typically contain from 5 to 10 to 25 to 50 to 100 to 500 to 1000 or more constitutional units. Polymers may be, for example, homopolymers, which contain multiple copies ofa single constitutional unit, or copolymers, which contain multiple copies of at least two dissimilar constitutional units, which units may be present in any of a variety of distributions including random, statistical, gradient, and periodic (e.g., alternating) distributions. The polymers for use in the present invention may have a variety of architectures, including cyclic, linear and branched architectures. Branched architectures include star-shaped architectures (e.g., architectures in which three or more chains emanate from a single branch point), comb architectures (e.g, architectures having a main chain and a plurality of side chains) and dendritic architectures (e.g., arborescent and hyperbranched polymers), among others. "Block copolymers" are polymers containing two or more differing polymer segments, for example, selected from homopolymer chains, and random and periodic copolymer chains.

In certain embodiments of the invention, implants in accordance with the invention are provided with coatings that comprise one or more electrically conductive polymers, which coatings may themselves be conductive in some instances. As defined herein a polymeric coating is conductive when it has a bulk conductivity of at least 10⁻⁴ S/cm.

It is known from several studies that coatings containing conductive polymers (e.g., polypyrrole, among others), are able to provide corrosion protection for metals. Moreover, even coatings with a low percentage of the conductive polymer have been shown to provide significant corrosion protection, as discussed below.

By way of background, many of the conductive polymers used in corrosion protection fall within the following classes: polyaniline and its derivatives and copolymers, poly(phenylene vinylene) and its derivatives and copolymers, and polyheterocycles and their derivatives and copolymers. Conductive polymers are commonly transformed from the insulating state to the conducting state through doping. Techniques for doping include, for example, (1) chemical doping by charge transfer, (2) electrochemical doping, (3) doping by acid-base chemistry (e.g., polyaniline undergoes this form of doping), (4) photodoping, and so forth.

For example, polyaniline (PANI) is normally prepared as the conductive polymerization techniques. By treatment with base the emeraldine base (EB) form can be obtained. The doping process used to increase and decrease the electrical conductivity of PANI is typically by protonation (doping) and de-protonation (de-doping). Examples of dopants include acids such as toluenesulfonic acid, perchloric acid, hydrochloric acid, and so forth. It is known to modify the polymer backbone of PANI through introduction of various functional groups such as alkyl, aryl, alkoxy, amino, and sulfonyl groups, among others, for example, in order to improve processing or to improve the chemical or physical characteristics of the material. PANI has been reported to work well in acidic environments but not in basic ones. This has been postulated to be due to the presence of the conductive emeraldine salt at acidic pH's, while at higher pH's (>7), the non-conductive emeraldine base is formed, which it is argued, does not provide adequate protection against corrosion. Nonetheless, both the doped and undoped forms of PANI have been shown to provide corrosion protection. For further information, see, e.g., P. Zarras, "Progress in using conductive polymers as corrosion-inhibiting coatings," Radiation Physics and Chemistry 68 (2003) 387-394 and the references cited therein. Examples of PANI copolymers include poly(aniline-co-2-anisidine) reported in G. Bereket et al., "Electrochemical synthesis and anti-corrosive properties of polyaniline, poly(2-anisidine), and poly(aniline-co-2-anisidine) films on stainless steel," Progress in Organic Coatings 54 (2005) 63-72, among others.

Among poly(phenylene vinylenes) (PPV), backbone-modified poly(bis-dialkylamino)phenylene vinylene) (BAM-PPV) has been used to form corrosion resistant conductive polymeric coatings. BAM-PPV is reported to be effective in neutral to basic conditions. For further information, see, e.g., N. Anderson, "A new conductive polymer as a replacement for chrome conversion coatings," 2003 Aerospace Coatings Removal and Coatings Conference, May 20-22, 2003, in Colorado Springs, CO, USA.

Polyheterocycles for use in conductive polymeric coatings include polypyrrole, polythiophene and their derivatives and copolymers. As with PANI and PPV above, it is known to modify the polymer backbone of these materials through the introduction of various functional groups (for instance, poly(3-alkyl pyrrole) and poly(3-alkyl thiophene) are examples of alkyl derivatives), and chemical or electrochemical polymerization techniques may be employed to form such polymers. For further information, see, e.g., P. Zarras, *supra,* and references cited therein.

Additional examples of conductive polymers include poly(2-anisidine) (see, e.g., G. Bereket et al., *supra)* and poly(thioflavin S) (see, e.g., N.F. Atta, "Electrochemical synthesis, characterization and some properties of a polymer derived from thioflavin S," European Polymer Journal 41 (2005) 3018-3025), among others.

Using the above and other conductive polymers, corrosion resistant coatings can be provided for use in the present invention, for example, by electropolymerization directly onto an underlying biodegradable metallic substrate or by chemical or electrochemical polymerization, which may be followed by further processing prior to coating onto an underlying biodegradable metallic region.

In addition to reducing corrosion in metallic materials, "conductive polymers have also been reported to encourage cell growth. In particular, polypyrrole has been observed to support the proliferation of endothelial cells. See, e.g., Gamer et al., "Polypyrrole-heparin composites as stimulus-responsive substrates for endothelial cell growth," J Biomed. Mater. Res. 1999 Feb; 44(2):121-9. Consequently, conductive polymer coatings on biodegradable metallic implants such as vascular stents may delay corrosion of the metallic structure while at the same time ensuring that substantial endothelial cell growth at the site of the implant is promoted.

As indicated above, biodegradable coatings are provided for corrosion protection in various embodiments of the invention. Although conductive polymers are generally not readily biodegradable, several strategies exist for creating biodegradable conductive polymeric coatings from the above and other conductive polymers.

In accordance with some strategies, for example, conducting oligomers may be formed form monomers of conductive polymers and connected via biodegradable linkages. In this regard, see, e.g., T.J. Rivers et al., "Synthesis of a Novel, Biodegradable Electrically Conducting Polymer for Biomedical Applications," Adv. Funct. Mater., 2002, 12, No. 1, January, 33-37, in which the synthesis and characterization of an electrically conducting, biodegradable polymer synthesized from conducting oligomers of pyrrole and thiophene is reported. The conducting oligomers are connected together via biodegradable ester linkages. The polymer has a conductivity of 10⁻⁴ S/cm and is soluble in THF, opening various options for solvent-based processing.

In other strategies, regions of conductive polymers are provided within a biodegradable polymer matrix. Such regions may be formed, for, example, by grinding or otherwise forming particles from a previously synthesized conductive polymer and later introduced to the biodegradable polymer(s). Such regions may also be formed in the presence of biodegradable polymer(s). For example, conductive polymer regions in the form of nanoparticles may be emulsion polymerized in a biodegradable polymer solution and the resulting composition coated onto a biodegradable metallic substrate. For instance, G. Shi et al., "A novel electrically conductive and biodegradable composite made of polypyrrole nanoparticles and polylactide," Biomaterials, 2004 Jun; 25(13):2477-88, have reported an electrically conductive biodegradable composite material made of poly(d,l-lactide) and polypyrrole nanoparticles, which has a surface resistivity as low as 1x10³ Ω/square. The material was prepared by emulsion polymerization of pyrrole in a poly(d,l-lactide) solution (in CHCl₃), followed by precipitation and casting onto a substrate. Of course application techniques other than casting, such as the coating techniques described elsewhere herein, may be employed to produce conductive polymeric regions of the desired form. See also A. Yfantis et al., "Novel corrosion-resistant films for Mg alloys," Surface and Coatings Technology 151-152 (2002) 400-404.

As another example, conductive polymer regions have been polymerized from the vapor phase within a porous biodegradable matrix. In this regard, see, e.g., Y. Wan et al., "Preparation and characterization of porous conducting poly(dl-lactide) composite membranes," Journal of Membrane Science 246 (2005) 193-201, which reports conductive biodegradable composite membranes, specifically, porous poly(dl-lactide), within which polypyrrole is incorporated by polymerizing pyrrole monomer from the vapor phase using FeCl₃ as oxidant. Using these and related techniques, a coating of biodegradable polymer may be applied to a biodegradable metallic region, rendered porous, and conducive polymer regions formed therein.

The degree of protection offered by conductive coatings has been observed to be influenced by the amount of conductive polymer within such composites. For example, in G. Shi et al., *supra,* a conductivity increase of six orders of magnitude was observed to accompany an increase in polypyrrole content from 1% to 17%. This may be used, for example, to optimize the corrosion resistance of an entire coating, or to provide a gradient or step-wise change in corrosion protection, if desired.

Suitable biodegradable polymers for use as matrix materials for conductive polymer regions may be selected, for example, from polyesters, polyanhydrides, and amino acid based polymers such as those set forth above, among others.

Various optional additives may be provided within the protective coatings of the invention. For example, in certain embodiments, a pH buffering agent may be added to offset pH changes associated with corrosion of the biodegradable metallic regions. For example, it is known that corrosion of magnesium and its alloys can result in an increase in pH, which may be, for instance, harmful to tissues surrounding an implant using from the same. pH buffering agents may be selected from any suitable pH buffering agent, and generally include those that are capable of maintaining the pH surrounding the implant in a range of from about 4 to about 8 standard pH units to avoid irritating or burning the surrounding biological tissue, for example, a blood vessel wall. Examples of pH buffering agents include anion- and cation-exchange resins, which may be selected, for instance, from the Amberlite® and Duolite® series of resins available from Rohm & Haas Corporation and the Dowex® series of resins available from Dow Corporation. Examples of suitable Amberlite® resins include Amberlite® IRP-64, Amberlite® IRP-68, Amberlite® IRP-88, Amberlite® CG-50 and Amberlyst® A21 resins. Examples of suitable Duolite® resins include Duolite® C-433, Duolite® A-368, and Duolite® A-392S resins. Examples of suitable Dowex® resins include Dowex® WGR, Dowex® WGR-Z, and Dowex® MWA-1 resins. In general, to aid in attaining the about 4 to about 8 pH range, the ion-exchange resin may be either weakly basic or acidic. The resin may be of fine particle size and is preferably of pharmaceutical grade. Resins of this type have been described for use in iontophoresis electrodes, for example, to avoid irritation or burning of the skin. See, e.g., U.S. Pat. No. 5,941,843 to Atanasoska et al.

As another example, chelating agents that capture metal cations associated with the breakdown of the biodegradable metals and metal alloys, may be employed (e.g., to remove metal species which may be somewhat toxic or which would participate in reactions that are undesirable, for example, reactions leading to a high pH environment, if not captured). For instance, biocompatible chelating agents that capture magnesium ions are known, including amino acids, chlorophyll, porphyrin, ethylenediaminetetraacetic acid (EDTA), diethylenetriaminepentaacetic acid (DTPA), ethyleneglycol-O,O'-bis(2-aminoethyl)-N,N,N',N'-tetraacetic acid (EGTA), N-(2-hydroxyethyl)ethylenediamine-N,N',N'-triacetic acid trisodium saIt (HEDTA), and polymer-chelating agent conjugates such as chitosan-EDTA and chitosan-DTPA conjugates, in which chelating agents such as EDTA and DTPA, among others, are provided along the polymer backbone (see, e.g., A. Bernkop-Schnürch, International Journal of Pharmaceutics 194, 2000, 1-13), among other chelating agents. Where provided within a biodegradable coating, these optional additive agents may form soluble metal complexes which are removed from the site of the implant and ultimately expelled from the body.

As yet another example, transport enhancing agents may be provided which enhance ion transport of metal cations that are associated with the breakdown of biodegradable metals and metal alloys through the corrosion resistant coating, such that they are released into the surrounding environment. For example, as described in D.A. Reece et al., *infra*, cyclodextrins are known to promote transport of Mg and Fe ions, among others.

As yet another example, imaging contrast agents (i.e., substances that enhance the image produced by medical diagnostic equipment) may be added to the coatings of the present invention. Among currently available contrast agents are magnetic resonance imaging (MRI) contrast agents, ultrasonic imaging contrast agents, and x-ray contrast agents, among others.

Ultrasound imaging contrast agents are materials that enhance the image produced by ultrasound equipment. Ultrasonic imaging contrast agents introduced into the compositions of the present invention can be, for example, echogenic (i.e., materials that result in an increase in the reflected ultrasonic energy) or echolucent (i.e., materials that result in a decrease in the reflected ultrasonic energy). Examples include microparticles/microspheres of calcium carbonate, hydroxyapatite, silica, poly(lactic acid), and poly(glycolic acid). Microbubbles may also be used as ultrasonic imaging contrast agents, as is known in the imaging art.

For contrast-enhanced MRI, it is desirable that the contrast agent have a large magnetic moment, with a relatively long electronic relaxation time. Based upon these criteria, contrast agents such as Gd(III), Dy(III), Mn(II) and Fe(III) have been employed. Gadolinium(III) has the largest magnetic moment among these three and is, therefore, a widely-used paramagnetic species to enhance contrast in MRI. Chelates of paramagnetic ions such as Gd-DTPA (gadolinium ion chelated with the ligand diethylenetriaminepentaacetic acid) have been employed as MRI contrast agents.

To be visible under x-ray (e.g., by x-ray fluoroscopy), devices and/or compositions are typically rendered more absorptive of x-rays than the surrounding tissue (i.e., they are rendered becoming more radiopaque). Examples of radiopaque agents include metals, metal salts and oxides, and iodinated compounds. More specific examples of such contrast agents include gold, tungsten, platinum, tantalum, iridium, or other dense metal, barium sulfate, bismuth subcarbonate, bismuth trioxide, bismuth oxychloride, metrizamide, iopamidol, iothalamate sodium, iodomide sodium, and meglumine.

As a specific example, both magnesium and iron possesses a low radiopacity due to their low atomic numbers. Thus placing two adjacent stents made of these metals (or stents made of their alloys, particularly those with additional constituents having low atomic numbers) within the same procedure may be problematic due to the invisibility of the first-placed stent under x-ray imaging. It would be therefore be advantageous in certain embodiments to provide these biodegradable stents with a radiopaque coating, for instance, a coating that contains gold particles.

For further information on imaging contrast agents, see, e.g., U.S. Patent Application No. 2003/0100830 entitled "Implantable or insertable medical devices visible under magnetic resonance imaging,".

Other optional additives include therapeutic agents. "Therapeutic agents", "pharmaceuticals," "pharmaceutically active agents", "drugs" and other related terms may be used interchangeably herein and include genetic therapeutic agents, non-genetic therapeutic agents and cells. Therapeutic agents may be used singly or in combination. Therapeutic agents may be, for example, nonionic or they may be anionic and/or cationic in nature.

Exemplary non-genetic therapeutic agents for use in connection with the present invention include: (a) anti-thrombotic agents such as heparin, heparin derivatives, urokinase, and PPack (dextrophenytalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluomuracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) beta-blockers, (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Several specific non-genetic therapeutic agents include paclitaxel (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Exemplary genetic therapeutic agents for use in connection with the present invention include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor a and β, platelet-derived endothelial growth factor, platelet-derived growth factor, tumor necrosis-factor a, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP), SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include Cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes; cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine; (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation-pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (I) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyan ionic agents (heparin, fucoidin), decorin, and TGF-S antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tycphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluoroumcil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, protessome inhibitors, angiogenesis inhibitors (e.g., endostatin, angiostatin and squalamine), raparnycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925 assigned to NeoRx Corporation.

A wide range of therapeutic agent loadings can be used in connection with the dosage forms of the present invention, with the pharmaceutically effective amount being readily determined by those of ordinary skill in the art and ultimately depending, for example, upon the condition to be treated, the nature of the therapeutic agent, the tissue into which the dosage form is introduced, and so forth.

As indicated above, in some embodiments of the invention, corrosion resistant coatings are configured to result in reduced corrosion in certain areas of the implants relative to other areas. For this purpose, some areas of the implants may be provided with coating materials, while others are not. Moreover, coating materials may be provided which provide differing corrosion protection, for example, due to a difference in the composition of the material making up the coating, due to a difference in the thickness of the coating material, and so forth. In this regard, the coating composition and/or thickness may change abruptly (e.g., in a stepwise fashion) and/or gradually along the surface of the implant.

Using a biodegradable metallic vascular stent formed from magnesium or a magnesium alloy as a specific example, as indicated above, such alloys are known to create a high pH (highly basic) environment in the areas where corrosion is occurring. With such a stent, corrosion may be preferentially promoted on the inside (luminal) surface (e.g., so that the corrosion products may be diluted and removed by the blood, rather than concentrating at the blood vessel wall where they may cause damage) in a number of ways, including the following: (a) providing no coating on the inside surface of the stent and a coating on the outside surface, (b) providing a thinner coating on the inside surface of the stent relative to the coating on the outside surface, (c) providing a first biodegradable coating material on the inside surface and a second, more slowly degrading, biodegradable coating material on the outside surface, (d) providing a biodegradable coating material on the inside surface and a biostable coating material on the outside surface, and so forth.

With regard to item (d) in the prior paragraph, among other embodiments, it is noted that a benefit of using biodegradable metals in stents is a reduction In the rigidity of the implant once it has served its function (e.g., in the case ofa vascular stent, after the patency of a blood vessel is restored). So long as a suitable flexible, biocompatible, biostable polymeric coating is employed (for example, a vinylaromatic/alkylene copolymer, such as the styrene-isobutylene copolymers described above, among others), this benefit may be achieved without significant adverse effects (e.g., without significant inflammation due to presence of the biostable polymer material in the body).

As another example, it may be desirable to biodegrade a stent along its length, much like the disappearance of a candle that bums at one or both ends. This may be implemented in a number of ways, including providing a progressively thicker (or thinner) coating along the length of the device, providing a gradient in coating composition along the length of the device, and so forth.

Corrosion resistant polymeric coatings may be formed using any of a variety of techniques depending upon the polymer or polymers making up the coatings, including, for example, physical vapor deposition, chemical vapor deposition, electrochemical deposition (e.g., for conductive polymers such as those described below), layer-by-layer techniques (discussed in detail below), and coating techniques based on the application of liquid polymer compositions, examples of which include polymer melts (e.g., where polymers having thermoplastic characteristics are employed), polymer solutions (e.g., where the polymers that are employed are dissolvable in an aqueous or organic solvents), and curable polymer systems (e.g., systems which undergo chemical cure, and systems that cure upon exposure to radiation, including UV light and heat), among other techniques.

Such liquid polymer compositions may be advantageous where it is desired to include one or more optional additives, such as pH buffering agents, chelating agents, transport enhancing agents, imaging contrast agents, therapeutic agents, and so forth, ins the coatings. Liquid polymer compositions may also be applied using a variety of application techniques, including dip coating, spin coating, spray coating, coating with an applicator (e.g., by roller or brush), web coating, stamping, screen printing, and ink jet printing, among other methods.

As indicated conductive polymers are commonly provided with one or more charged doping species to increase conductivity. In some embodiments, charged species may be included to increase conductivity. Charged species may also be included to provide functionality in addition to, or other than, increasing conductivity, and may be provided in both conductive and nonconductive polymer coatings in accordance with the invention.

For example, charged species may be provided to inhibit corrosion. See, e.g., G. Paliwoda-Porebska et al., "On the development of polypyrrole coatings with self-healing properties for iron corrosion protection," Corrosion Science 47 (2005) 3216-3233, where it is demonstrated that polypyrrole, doped with [PMo₁₂O₄₀]³⁻ anions, releases the same when the potential at the interface decreases, for example, as a consequence of metal corrosion at the location of a coating defect. Upon decomposition, [PMo₁₂O₄₀]³⁻ anions yield Mo₄O²⁻ and HPO₄²⁻ anions. MoO₄²⁻ is an inhibitor of iron dissolution and is able to passivate the defect in the coating. Thus, inhibitor anions are preferentially released at active defects, thereby providing "intelligent" corrosion inhibition.

Charged species may also be provided to modulate drug release. For example, certain charged species may complex with, and increase the water solubility of, a given therapeutic agent.

Other examples of charged species are polyoxometallates (POMs). POMs are a large class of nanosized, anionic, metal and oxygen containing molecules. Polyoxometallates have been synthesized for many years (the first known synthesis dates back to 1826), they readily self assemble under appropriate conditions (e.g., acidic aqueous media), and they are quite stable. POMs comprise one or more types of metal atoms, sometimes referred to as addenda atoms (commonly molybdenum, tungsten, vanadium, niobium, tantalum or a mixture of two or more of these atoms), which with the oxygen atoms form a framework (sometimes referred to as the "shell" or "cage") for the molecule. More specific examples include V^{V}, Nb^{V}, Mo^{VI} and W^{VI}, among others. Some POMs further comprise one or more types of central atoms, sometimes referred to as heteroatoms, which lie within the shell that is formed by the oxygen and addenda atoms. A very wide variety of elements (i.e., a majority of elements in the periodic table) may act as heteroatoms, with some typical examples being P⁵⁺, As⁵⁺, Si⁴⁺, Ge⁴⁺, B³⁺, and so forth. In certain cases, one or more of the oxygen atoms within the POM is/are substituted by S, F, Br and/or other p-block elements. Materials for forming POMs may be obtained, for example, from Sigma Aldrich and Goodfellow Corp., among other sources.

POMs are known, for example, to have antitumor and antiviral behavior. See, e.g., A. Ogata et al., "A novel anti-tumor agent, polyoxomolybdate induces apoptotic cell death in AsPC-1 human pancreatic cancer cells," Biomedicine & Pharmacotherapy 59 (2005) 240-244; "Study of Some Polyoxometallates of Keggin's Type as Potential Antitumour Agents," Jugoslov Med Biohem 2004, 23; Lan Ni et al., "Cellular localization of antiviral polyoxometalates in J774 Macrophages," Antiviral Research 32 (1995) 141-148.

Being charged, POMs may be used to modulate drug release, for example, by forming complexes with charged therapeutic agents.

POMs are known to act as templates for the growth of conductive polymer nanoparticles, which may be, for example, grown in situ, grown and then dispersed in a polymer matrix, grown and deposited in a layer-by-layer self-assembly scheme, and so forth. See, e.g., L. Liu et al., "Characteristics of polypyrrole (PPy) nano-tubules made by templated ac electropolymerization," European Polymer Journal 41 (2005) 2117-2121. See also F. Wang et al., "Polyaniline microrods synthesized by a polyoxometalates/poly(vinyl alcohol) microfibers template" Materials Letters 59 (2005) 3982-3985, in which polyaniline microrods were obtained by a microfiber template method. The average diameter of the polyaniline microrods was between 200 and 250 nm.

POMs are also known to assist in the formation of conductive polymer films. See, e.g., X. Zou, "Preparation of a phosphopolyoxomolybdate P2Mo18O626- doped polypyrrole modified electrode and its catalytic properties," Journal of Electroanalytical Chemistry 566 (2004) 63-71 and S.A. Cheng et al., Synthetic Metals, 129 (2002) 53-59, in which polymer-POM hybrid materials, specifically, POM-doped polypyrrole films, were formed by the electropolymerization of pyrrole in the presence of POMs. In both cases it was noted that Mo-substituted POMs are able to stimulate/catalyze the polymerization of pyrrole.

Alternatively, preformed POMs may simply be dispersed in polymer solutions, for example conductive or non-conductive polymer solutions, using suitable dispersion techniques such as ultrasound, among others. See, e.g., W. Feng et al., "Sonochemical preparation of photochromic nanocomposite thin film based on polyoxometalates well dispersed in polyacrylamide," Journal of Solid State Chemistry 169 (2002) 1-5.

As indicated above, the polymeric coatings of the invention may also include one or more optional additives, whether charged or uncharged, such as pH buffering agents, chelating agents, imaging contrast agents, therapeutic agents, transport enhancing agents, and so forth. Regarding the latter, see, e.g., D.A. Reece et al., "Metal transport studies on inherently conducting polymer membranes containing cyclodextrin dopants," Journal of Membrane Science 249 (2005) 9-20, in which conducting polymer films are prepared electrochemically using aqueous solutions containing polypyrrole (PPy) and either sulfated alpha-cyclodextrin (alpha -CDS) or sulfated beta-cyclodextrin(beta-CDS) as a dopant. Electrical conductivities of the films were found to be 0.7 S cm⁻¹ and 0.4 S cm⁻¹, respectively. In general, the beta-CDS films were more permeable to transport of metal ions (including magnesium ions) than were the alpha-CDS films.

As indicated above, polymeric coatings in accordance with the present invention may also be created by processes, commonly known as layer-by-layer (LbL) techniques, in which substrates are coated using charged materials via electrostatic self assembly. The resulting self-assembled charged layers then act as a time-limited corrosion protective coating.

In a typical layer-by-layer process, multilayer growth proceeds through sequential steps, in which a substrate is immersed in solutions of cationic and anionic species, frequently with intermittent rinsing between steps. In this way, a first layer having a first surface charge is typically deposited (or adsorbed) on an underlying substrate, followed by a second layer having a second surface charge that is opposite in sign to the surface charge of the first layer, and so forth. The charge on the outer layer is reversed upon deposition of each sequential layer. Commonly, 5 to 10 to 25 to 50 to 100 to 200 or more layers are applied in this technique, depending, for example, on the degree of corrosion protection desired.

multilayer regions created using layer-by-layer self-assembly generally include one or more types of polyelectrolytes as ionic species. As used herein, "polyelectrolytes" are polymers having multiple (e.g., 5 to 10 to 25 to 50 to 100 to 250 to 500 to 1000 or more) charged groups (e.g., ionically dissociable groups that provide cations and anions).

Frequently, the number of charged groups is so large that the polymers are soluble in polar solvents (including water) when in ionically dissociated form (also called polyions). Depending on the type of dissociable groups, polyelectrolytes may be classified as polyacids and polybases. When dissociated, polyacids form polyanions, with protons being split off. Examples of polyacids are polyphosphoric acids, polyvinylsulfuric acids, polyvinylsulfonic acids, polyvinylphosphonic acids and polyacrylic acids. Examples of the corresponding salts, which are also called polysalts, are polyphosphates; polyvinylsulfates, polyvinylsulfonates, polyvinylphosphonates and polyacrylates. Polybases contain groups which are capable of accepting protons, e.g., by reaction with acids, with a salt being formed. Examples of polybases having dissociable groups within their backbone and/or side groups are polyallylamine, polyethylimine, polyvinylamine and polyvinylpyridine. By accepting protons, polybases form polycations.

Some polyelectrolytes have both anionic and cationic groups, but nonetheless have a net negative charge, for example, because the anionic groups outnumber the cationic groups, or have a net positive charge, for example, because the cationic groups outnumber the anionic groups. In this regard, the net charge of a particular polyelectrolyte may change with the pH of its surrounding environment. Polyelectrolytes containing both cationic and anionic groups are generally categorized herein as either polycations or polyanions, depending on which groups predominate.

Thus, as defined herein, the term "polyelectrolyte" embraces a wide range of species, including polycations and their precursors (e.g., polybases, polysalts, etc.), polyanions and their precursors (e.g., polyacids, polysalts, etc.), polymers having multiple anionic and cationic groups (e.g., polymers having multiple acidic and basic groups such as are found in various proteins), ionomers (polyelectrolytes in which a small but significant proportion of the constitutional units carry charges), and so forth.

Linear or branched polyelectrolytes may be used in some embodiments. Using branched polyelectrolytes can lead to less compact polyelectrolyte multilayers having a higher degree of wall porosity, which may result in increased corrosion. Polyelectrolyte molecules may be crosslinked within or/and between the individual layers in some embodiments (e.g., by crosslinking amino groups with aldehydes, by heating, etc.), for example, to increase stability and thus lengthen corrosion protection.

Specific examples of suitable polycations may be selected, for instance, from the following: polyamines, including polyamidoamines, poly(amino methacrylates) including poly(dialkylaminoalkyl methacrylates) such as poly(dimethylaminoethyl methacrylate) and poly(diethylaminoethyl methacrylate), polyvinylamines, polyvinylpyridines including quaternary polyvinylpyridines such as poly(N-ethyl-4-vinylpyridine), poly(vinylbenzyltrimethylamines), polyallylamines such as poly(allylamine hydrochloride) (PAH) and poly(diallyldialklylamines) such as poly(diallyldimethylammonium chloride), spermine, spermidine, hexadimethrene bromide (polybrene), polyimines including polyalkyleneimines such as polyethyleneimines, polypropyleneimines and ethoxylated polyethyleneimines, basic peptides and proteins, including histone polypeptides and homopolymer and copolymers containing lysine, arginine, ornithine and combinations thereof including poly-L-lysine, poly-D-lysine, poly-L,D-lysine, poly-L-arginine, poly-D-arginine, poly-D,L-arginine, poly-L-omithine, poly-D-omithine, and poly-L,D-ornithine, gelatin, albumin, protamine and protamine sulfate, and polycationic polysaccharides such as cationic starch and chitosan, as well as copolymers; derivatives and combinations of the preceding, among various others.

Specific examples of suitable polyanions may be selected, for instance, from the following: polysulfonates such as polyvinylsulfonates, poly(styrenesulfonates) such as poly(sodium styrenesulfonate) (PSS), sulfonated poly(tetrafluoroethylene), sulfonated polymers such as those described in U.S. Patent No. 5,840,387, including sulfonated styrene-ethylene/butylene-styrene triblock copolymers, sulfonated styrenic homopolymers and copolymers such as a sulfonated versions of the polystyrene-polyolefin copolymers described in U.S. Patent No. 6,545,097 to Pinchuk et al., which polymers may be sulfonated, for example, using the processes described in U.S. Patent No. 5,840,387 and U.S. Pat. No. 5,468,574, as well as sulfonated versions of various other homopolymers and copolymers, polysulfates such as polyvinylsulfates, sulfated and non-sulfated glycosaminoglycans as well as certain proteoglycans, for example, heparin, heparin sulfate, chondroitin sulfate, keratan sulfate, dermatan sulfate, polycarboxylates such as acrylic acid polymers and salts thereof (e.g., ammonium, potassium, sodium, etc.), for instance, those available from Atofina and Polysciences Inc., methacrylic acid polymers and salts thereof (e.g., EUDRAGIT, a methacrylic acid and ethyl acrylate copolymer), carboxymethylcellulose, carboxymethylamylose and carboxylic acid derivatives of various other polymers, polyanionic peptides and proteins such as glutamic acid polymers and copolymers, aspartic acid polymers and copolymers, polymers and copolymers of uronic acids such as mannuronic acid, galatcuronic acid and guluronic acid, and their salts, for example, alginic acid and sodium alginate, hyaluronic acid, gelatin, and carrageenan, polyphosphates such as phosphoric acid derivatives of various polymers, polyphosphonates such as polyvinylphosphonates, polysulfates such as polyvinylsulfates, as well as copolymers, derivatives and combinations of the preceding, among various others.

In certain embodiments of the invention, the polyelectrolytes selected are biodegradable polyelectrolytes (which may act as therapeutic agents as well). Examples of biodegradable polyelectrolytes include suitable members of those set forth above, including, for example, biodegradable polycations such as chitosan, protamine sulfate, gelatin, spermidine, and albumin, among many others, and biodegradable polyanions such as heparin, sodium alginate, gelatin [gelatin is an amphiphilic polymer, hence it fits in both categories depending how it is being prepared], hyaluronic acid, carrageenan, and chondroitin sulfate, among many others.

Although multilayer polyelectrolyte films are permeable to water, these films, which are commonly less than a micron in thickness, have been shown to have a significant effect on the corrosion rate. For example, T.R. Farhat et al., "Corrosion Control Using Polyelectrolyte Multilayers," Electrochemical and Solid-State Letters Volume 5, Issue 4, pp. B13-B15, April 2002, have reported that the corrosion of stainless steel under anodic conditions in salt solutions was strongly suppressed by polyelectrolyte multilayers applied using the LbL deposition method. Effective corrosion control was observed for both hydrophilic and hydrophobic layers, despite the significant water content and ion permeability of the thin films. An explanation of the reduced corrosion rate is based on the reduced chemical activity of water, due to its being bound by the polyelectrolyte ion pairs. This also explains why both hydrophilic as well as hydrophobic LBL films have similar protective results. The higher the electrostatic attraction between the anionic\cationic pairs the stronger water is chemically bound.

Regardless of the mechanism of operation, attractive features of multilayer polyelectrolyte coatings include their corrosion protection ability and their ability to cover medical implants having complex 3D contours such as stents. In this regard, such coatings bond to the underlying metal substrate in a manner which excludes air pockets, which are usually found in other polymer-based protective coatings. Such pockets are a pitting haven and allow for a quick start in the corrosion process. In addition to adhering closely to the substrate, polyelectrolyte coatings are compliant as well, allowing stents to be expanded without causing cracks in the coatings. Cracks in conventional corrosive protection films cause corrosion virtually immediately.

The use of biodegradable polyelectrolytes enable one to suppress corrosion for a predefined time, depending on the number of polyelectrolyte layers as well as the choice of polycations and polyanions. Coatings made from these are fully biodegradable in that the polyelectrolytes forming them are broken down in vivo.

Charged layers deposited in the LbL process may also optionally include one or more charged therapeutic agents. By "charged therapeutic agent" is meant a therapeutic agent that has an associated charge. For example, a therapeutic agent may have an associated charge because it is inherently charged (e.g., because it has acidic and/or or basic groups which may be in salt form). A few examples of inherently charged cationic therapeutic agents include amiloride, digoxin, morphine, procainamide, and quinine, among many others. Examples of anionic therapeutic agents include heparin and DNA, among many others.

A therapeutic agent may have an associated charge because it has been chemically modified to provide it with one or more charged functional groups. For instance, conjugation of water insoluble or poorly soluble drugs (e.g., anti-tumor agents such as paclitaxel, among others) to hydrophilic polymers has recently been conducted in order to solubilize the drug (and in some cases to improve tumor targeting and reduce drug toxicity). Similarly non-polymeric cationic or anionic derivates of water insoluble or poorly soluble drugs have also been developed.

Taking paclitaxel as a specific example, various cationic forms of this drug are known, including paclitaxel N-methyl pyridinium mesylate and a polyelectrolyte in which paclitaxel is conjugated with N-2-hydroxypropyl methyl amide, as are various anionic forms of paclitaxel, including polyelectrolyte forms such as paclitaxel-poly(1-glutamic acid), paclitaxel-poly(1-glutamic acid)-PEO. See, e.g., U.S. Patent No. 6,730,699; Duncan et al., Journal of Controlled Release 74 (2001)135; Duncan, Nature Reviews/Drug Discovery, Vol. 2, May 2003, 347; Jaber G. Qasem et al, AAPS PharmSciTech 2003, 4(2) Article 21. In addition to these, U.S. Patent No. 6,730,699, also describes polyelectrolyte forms of paclitaxel in which paclitaxel is conjugated to various charged polymers including poly(d-glutamic acid), poly(dl-glutamic acid), poly(l-aspartic acid), poly(d-aspartic acid), poly(dl-aspartic acid), poly(l-lysine), poly(d-lysine), poly(dl-lysine), copolymers of the above listed polyamino acids with polyethylene glycol (e.g., paclitaxel-poly(l-glutamic acid)-PEO), as well as poly(2-hydroxyethyl 1-glutamine), chitosan, carboxymethyl dextran, hyaluronic acid, human serum albumin and alginic acid. Still other forms of paclitaxel include carboxylated forms such as 1'-malyl paclitaxel sodium salt (see, e.g. E. W. DAmen et al., "Paclitaxel esters of malic acid as prodrugs with improved water solubility," Bioorg Med Chem., 2000 Feb, 8(2), pp. 427-32). Polyglutamate paclitaxel, in which paclitaxel is linked through the hydroxyl at the 2' position to the A-carboxylic acid of the poly-L-glutamic acid (PGA), is produced by Cell Therapeutics, Inc., Seattle, WA, USA. (The 7 position hydroxyl is also available for esterification.) This molecule is said to be cleaved in vivo by cathepsin B to liberate diglutamyl paclitaxel. In this molecule, the paclitaxel is bound to some of the carboxyl groups along the backbone of the polymer, leading to multiple paclitaxel units per molecule. For further information, see, e.g., R. Duncan et al., "Polymer-drug conjugates, PDEPT and PELT: basic principles for design and transfer from the laboratory to clinic," Journal of Controlled Release 74 (2001) 135-146, C. Li, "Poly(L-glutamic acid anticancer drug conjugates," Advanced Drug Delivery Reviews 54 (2002) 695-713; Duncan, Nature Reviews/Drug Discovery, Vol. 2, May 2003, 347; Qasem et al, AAPS PharmSciTech 2003, 4(2) Article 21; and U.S. Patent No. 5,614,549.

A therapeutic agent may also have an associated charge because it is associated with a charged particle, which may in turn participate in the layer-by-layer assembly process. For example, a therapeutic agent may be attached to a charged nanoparticle (i.e., a charged particle having a major cross-sectional dimension of 100 nm or less, for example, a spherical particle or a rod-shaped particle having a diameter of 100 nm or less, a ribbon shaped particle having a thickness of 100 nm or less, etc.), or it may be encapsulated within a charged particle, for example, encapsulated within a charged nanocapsule (such as a single layer or multilayer nanocapsule), or it may be provided within a charged micelle, among other possibilities. Other examples include protein-bound paclitaxel particles such as albumin-bound paclitaxel nanoparticles, e.g., ABRAXANE. A therapeutic agent may be provided within a charged capsule, for example, using LbL techniques such as those described above and in commonly assigned U.S. Patent App. Pub. No. 2005/0129727.

In addition to therapeutic agents, other agents may be encapsulated within polyelectrolyte multilayer shells, for example, anticorrosion agents such as salt of the [PMo₁₂O₄₀]³⁻ anions described above, among others.

Further charged species which may participate in the LbL process include pH buffering agents such as the ion exchange resins (e.g., insoluble polyelectrolyte networks that are able to exchange counterions, specifically, cations or anions, with the ionic components of a solution) and imaging contrast agents. With respect to the latter, one may incorporate polyelectrolyte coated gold nanoparticles to increase temporarily the radiopacity of the implant. Gold nanoparticles are biocompatible and can be incorporated directly into the polylectrolyte coating as a charged layer. As the coating degrades, the gold particles enter the bloodstream and are removed from the body. For examples of polyelectrolyte coated gold nanoparticles see G. Schneider et al., "From Functional Core/Shell Nanoparticles Prepared via Layer-by-Layer Deposition to Empty Nanospheres," Nano Letters, 4 (10), 1833 -1839, 2004, who report that gold nanoparticles can easily be coated using LbL (LbL) deposition. Such nanoparticles are charged and can be assembled in the LbL process. For example, Yanjing Liu et al., "Layer-by-layer ionic self assembly of Au colloids into multilayer thin-fims with bulk metal conductivity," Chemical Physics Letters 298 (1998) 315-319, report that colloidal Au nanoparticles, each encapsulated by polyelectrolytes, have been self-assembled into multilayer films in a LbL fashion, alternating layers of positively charged, encapsulated nanoparticles with a negatively charged polyelectrolyte.

Other examples of charged species which may be assembled in the LbL process includes polyoxometallates (POMs), which are described in more detail above. As noted above, POMs are known to have antitumor and antiviral behavior. Moreover, POMs are also known to act as templates for the growth of conductive polymer nanoparticles, for example, polypyrrole and polyaniline nanoparticles. The resulting nanoparticles are charged and thus may participate in the LbL assembly process. Being conductive the resulting nanoparticles may also enhance the anti-corrosion effect of the coating. As noted above, polypyrrole is also known to promote endothelial cell growth. For examples of the use of POMs in LbL assembly, see, e.g., J. Zhang et al., Materials Chernistry and Physics 90 (2005) 47-52, in which a multilayer film constructed of an anionic Dawson-type POM and a diazo resin was prepared (which film could be stabilized by the photoinduced interaction between the POM and diazo resin if desired) and Y. Wang et al., "Self-assembled multilayer films based on a Keggin-type potyoxometatate and polyaniline," Journal of Colloid and Interface Science 264 (2003) 176-183, in which a multilayer film constructed of a Keggin-type POM and cationic polyaniline (PAN, emeraldine base) was prepared. With respect to the latter, the multilayer films were conductive, exhibiting conductivities on the order of 10⁻³ S cm⁻¹. Thus films may be formed, which are both electroconductive and electrostatically self-assembled.

Further examples of charged conductive polymer species which may participate in LbL assembly techniques include the polypyrrole-heparin composite polymers described in Garner et al. *supra.* See also C. Sun et al., "Fabrication of a multilayer film electrode containing porphyrin and its application as a potentiometric sensor of iodide ion," Talanta 46 (1998) 15-21 in which a low resistance, ion-selective electrode was produced using a LbL deposition techniques in wherein water-soluble porphyrin is alternatively deposited with water-soluble polypyrrole on a 2-aminoethanethiol modified metallic (e.g., silver) substrate. As noted above, porphyrin is a metal chelating agent which forms complexes with metals ions such as Mg ions.

The LbL process generally begins by providing a charged substrate. Certain substrates are inherently charged and thus readily lend themselves to LbL assembly techniques. To the extent that the substrate does not have an inherent net surface charge, a surface charge may nonetheless be provided. For example, in the present invention, the biodegradable metallic regions to be coated are conductive, and a surface charge may be provided by applying a suitable electrical potential to the same.

As another example, charged groups may be introduced by binding charged compounds to the biodegradable metallic surfaces, for example, through covalent interactions or non-covalent interactions such as van der Waals interactions, hydrogen bonding, hydrophilic/hydrophobic interactions and/or other interactions between the substrate and the charged compounds.

For instance, a surface charge may be provided on a substrate by exposing the substrate to a charged amphiphilic substance. Amphiphilic substances include any substance having hydrophilic and hydrophobic groups. Where used, the amphiphilic substance should have at least one electrically charged group to provide the substrate surface with a net electrical charge. Therefore, the amphiphilic substances that are used herein can also be referred to as ionic amphiphilic substances.

Amphiphilic polyelectrolytes are used as ionic amphiphilic substances in some embodiments. For example, a surface charge may be provided on a substrate by adsorbing polycations (for example, selected from polyethylenimine (PEI), protamine sulfate, polyallylamine, polydiallyldimethylammonium species, chitosan, gelatin, spermidine, and albumin, among others) or by adsorbing polyanions (for example, selected from polyacrylic acid, sodium alginate, polystyrene sulfonate (PSS), eudragit, gelatin, hyaluronic acid, carrageenan, chondroitin sulfate, and carboxymethylcellulose, among others) to the surface of the substrate as a first charged layer. PEI is commonly used for this purpose, as it strongly promotes adhesion to a variety of substrates. This process has been demonstrated on glass substrates using charged polymeric (polyelectrolyte) materials. See, e.g., "Multilayer on solid planar substrates," Multi-layer thin films, sequential assembly of nanocomposite materials, Wiley-VCH ISBN 3-527-30440-1, Chapter 14; and Hau, Winky L. W. et al. "Surface-chemistry technology for microfluidics," J. Micromech. Microeng. 13 (2003) 272-278. See also, Y. Wang et al. *supra* in which a PEI/PSS precursor LbL film was deposited onto a cleaned substrate, prior to assembly of the desired POM/PAN LbL film.

The technology of self-assembled, charged layers allows one to define the coating composition and thickness on a local scale. One could, therefore, design the corrosion process to be specific for each region of the implant (e.g., each stent element). As a specific example, the ends of a stent may be coated with 10 layers, while the middle section may be coated with 20 layers, in which case the ends will start corroding before the middle section. As the LbL coating process can be very straightforward, for example, based on repeated dipping cycles, dipping the stent to 2/3 of its length to apply the first 10 coating layers and reversing the orientation of the stent for the next 10 layers will double she amount of layers on the middle 1/3 part. This is, of course, a very simple example. On the other hand, one can create very complex polyelectrolyte coating patterns using techniques such as spraying techniques, roll and brush coating techniques, ink jet techniques, and micro-polymer stamping techniques. For the latter, see, e.g., S. Kidambi et al., "Selective Depositions on Polyelectrolyte Multilayers: Self-Assembled Monolayers ofm-dPEG Acid as Molecular Templates" J. Am. Chem. Soc 126, 4697-4703, 2004.

Certain LbL films are also known which close at high pH (see, e.g., A. Alexei et al., "Polyelectrolyte multilayer capsules as vehicles with tunable permeability," Advances In Colloid and Interface Science 111 (2004) 49-61), thereby offering the potential to create self-regulating coatings, particularly for biodegradable metallic materials such as magnesium and magnesium alloys, which produce high pH products during the corrosion process.

## Claims

1. An implantable stent comprising
- a biodegradable magnesium or magnesium alloy metallic region (100) and
- a biodegradable polymeric corrosion resistant coating (110) over the biodegradable metallic region (100) that slows the rate of corrosion of the biodegradable metallic region (100) upon implantation into a subject, wherein said corrosion resistant coating (110) comprises an electrically conductive polymer.

2. The implantable stent of claim 1, wherein the biodegradable metallic region corresponds to an entire implantable stent, or to a component of an implantable stent.

3. The implantable stent of claim 1, comprising a plurality of said biodegradable metallic regions, or comprising a plurality of said corrosion resistant coatings, or wherein said corrosion resistant coating is provided over only a portion of said biodegradable metallic region, or wherein said corrosion resistant coating varies in thickness along a surface of said implantable stent, or wherein said corrosion resistant coating varies in composition along a surface of said implantable stent.

4. The implantable stent of claim 1, wherein said polymeric coating comprises an additive selected from a pH buffering agent, a chelating agent, an anticorrosion agent, a transport enhancing agent, and combinations thereof.

5. The implantable stent of claim 1, wherein said corrosion resistant coating comprises an imaging contrast agent, preferably comprises gold particles.

6. The implantable stent of claim 1, wherein said corrosion resistant coating comprises a therapeutic agent.

7. The implantable stent of claim 1, wherein said electrically conductive polymer is selected from homopolymers, copolymers, and derivatives of aniline, pyrrole, thiophene, phenylene vinylene, anisidine, and thioflavin as well as combinations of the same.

8. The implantable stent of claim 1, wherein said electrically conductive polymer is biodegradable.

9. The implantable stent of claim 1, wherein said corrosion resistant coating comprises regions of said electrically conductive polymer within a biodegradable polymeric matrix, preferably wherein said biodegradable polymer matrix comprises a biodegradable polymer selected from polyesters, polyanhydrides, and amino acid based biodegradable polymers.

10. The implantable stent of claim 1, wherein said corrosion resistant coating comprises a charged dopant species.

11. The implantable stent of claim 1, wherein said corrosion resistant coating comprises a charged species selected from a corrosion inhibitor, a therapeutic agent, and combinations thereof

12. The implantable stent of claim 1, wherein said corrosion resistant coating comprises charged particles, preferably wherein said charged particles are selected from particles that comprise a species selected from conductive polymers, radiopaque agents, polyoxometallates, therapeutic agents, anticorrosion agents, and combinations of the same.

13. The implantable stent of claim 1, wherein said corrosion resistant coating comprises (a) a plurality of positively charged layers comprising a positively charged polyelectrolyte, (b) a plurality of negatively charged layers comprising a negatively charged polyelectrolyte, or (c) a plurality of positively charged layers comprising a positively charged polyelectrolyte and a plurality of negatively charged layers comprising a negatively charged polyelectrolyte.

14. The implantable stent of claim 13, wherein said corrosion resistant coating comprises from 10 to 200 layers, or wherein said corrosion resistant coating further comprises a layer that comprises charged particles preferably wherein said charged particles are selected from particles that comprise a species selected from electrically conductive polymers, radiopaque agents, polyoxometallates, therapeutic agents, anticorrosion agents, pH buffering agents, chelating agents, transport enhancing agents and combinations of the same, or comprising a polyelectrolyte that comprises an agent selected from radiopaque agents, therapeutic agents, anticorrosion agents, pH buffering agents, chelating agents, transport enhancing agents, and combinations thereof., or comprising a polyelectrolyte that comprises a conductive polymer, preferably wherein said conductive polymer is selected from polypyrrole, polyaniline, and combinations thereof, or wherein said corrosion resistant coating varies in porosity with changing pH, or wherein said corrosion resistant coating decreases in porosity with increasing pH, or wherein said corrosion resistant coating comprises a plurality of positively charged layers comprising a biodegradable positively charged polyelectrolyte and a plurality of negatively charged layers comprising a biodegradable negatively charged polyelectrolyte.

## Patentansprüche

1. Ein implantierbarer Stent umfassend
- einen biodegradierbaren, metallischen Magnesium- oder Magnesiumlegierung-Bereich (100) und
- eine biodegradierbare, polymerische, korrosionsresistente Beschichtung (110) über dem biodegradierbaren, metallischen Bereich (100), die die Korrosionsrate des biodegradierbaren, metallischen Bereichs verlangsamt nach der Implantation in ein Subjekt, wobei die korrosionsresistente Beschichtung (110) ein elektrisch leitfähiges Polymer umfasst.

2. Der implantierbare Stent nach Anspruch 1, wobei der biodegradierbare, metallische Bereich einem gesamten implantierbaren Stent oder einem Teil eines implantierbaren Stents entspricht.

3. Der implantierbare Stent nach Anspruch 1, eine Vielzahl der biodegradierbaren metallischen Bereiche umfassend oder eine Vielzahl der korrosionsresistenten Beschichtungen umfassend oder wobei die korrosionsresistente Beschichtung in der Dicke variiert entlang einer Oberfläche des implantierbaren Stents oder wobei die korrosionsresistente Beschichtung in der Zusammensetzung variiert entlang einer Oberfläche des implantierbaren Stents.

4. Der implantierbare Stent nach Anspruch 1, wobei die polymerische Beschichtung ein Additiv umfasst, ausgewählt aus einer pH Puffersubstanz, einem Chelatbildner, einer Antikorrosionssubstanz, einer transportsteigernden Substanz und Kombinationen davon.

5. Der implantierbare Stent nach Anspruch 1, wobei die korrosionsresistente Beschichtung ein bildgebendes Kontrastmittel umfasst, bevorzugt Goldpartikel umfasst.

6. Der implantierbare Stent nach Anspruch 1, wobei die korrosionsresistente Beschichtung ein Therapeutikum umfasst.

7. Der implantierbare Stent nach Anspruch 1, wobei das elektrisch leitfähige Polymer ausgewählt ist aus Homopolymeren, Copolymeren und Derivaten von Anilin, Pyrrol, Thiophen, Phenylen-Vinylen, Anisidin und Thioflavin genauso wie Kombinationen daraus.

8. Der implantierbare Stent nach Anspruch 1, wobei das elektrisch leitfähige Polymer biodegradierbar ist.

9. Der implantierbare Stent nach Anspruch 1, wobei die korrosionsresistente Beschichtung Bereiche des elektrisch leitfähigen Polymers innerhalb einer polymerischen Matrix umfasst, bevorzugt wobei die biodegradierbare Polymer-Matrix ein biodegradierbares Polymer umfasst, ausgewählt aus Polyestern, Polyanhydriden und auf Aminosäuren basierenden biodegradierbaren Polymeren.

10. Der implantierbare Stent nach Anspruch 1, wobei die korrosionsresistente Beschichtung eine geladene Dotierstoff-Art umfasst.

11. Der implantierbare Stent nach Anspruch 1, wobei die korrosionsresistente Beschichtung eine geladene Art umfasst, ausgewählt aus einem Korrosionsinhibitor, einem Therapeutikum und Kombinationen davon.

12. Der implantierbare Stent nach Anspruch 1, wobei die korrosionsresistente Beschichtung geladene Partikel umfasst, bevorzugt wobei die geladenen Partikel ausgewählt sind aus Partikeln, die eine Art umfassen, die ausgewählt ist aus leitfähigen Polymeren, röntgenopaken Substanzen, Polyoxometallaten, Therapeutika, Antikorrosionssubstanzen und Kombinationen davon.

13. Der implantierbare Stent nach Anspruch 1, wobei die korrosionsresistente Beschichtung umfasst (a) eine Vielzahl von positiv geladenen Schichten, die ein positiv geladenes Polyelektrolyt umfassen, (b) eine Vielzahl von negativ geladenen Schichten, die ein negativ geladenes Polyelektrolyt umfassen, oder (c) eine Vielzahl von positiv geladenen Schichten, die ein positiv geladenes Elektrolyt umfassen und eine Vielzahl von negativ geladenen Schichten, die ein negativ geladenes Elektrolyt umfassen.

14. Der implantierbare Stent nach Anspruch 13, wobei die korrosionsresistente Beschichtung 10 bis 200 Schichten umfasst, oder wobei die korrosionsresistente Beschichtung weiter umfasst eine Schicht, die geladene Partikel umfasst, bevorzugt wobei die geladenen Partikel ausgewählt sind aus Partikeln, die eine Art umfassen, die ausgewählt ist aus elektrisch leitfähigen Polymeren, röntgenopaken Substanzen, Polyoxometallaten, Therapeutika, Antikorrosionssubstanzen, pH Puffersubstanzen, Chelatbildnern, transportsteigernden Substanzen und Kombinationen derselben, oder ein Polyelektrolyt umfasst, dass eine Substanz umfasst, ausgewählt aus röntgenopaken Substanzen, Therapeutika, Antikorrosionssubstanzen, pH Puffersubstanzen, Chelatbildnern, transportsteigernden Substanzen und Kombinationen davon, oder ein Polyelektrolyt umfasst, dass ein leitfähiges Polymer umfasst, bevorzugt wobei das leitfähige Polymer ausgewählt ist aus Polypyrrol, Polyanilin und Kombinationen davon, oder wobei die Porosität der korrosionsresistenten Beschichtung sich ändert mit sich veränderndem pH, oder wobei die Porosität der korrosionsresistenten Beschichtung abnimmt mit steigendem pH, oder wobei die korrosionsresistente Beschichtung eine Vielzahl von positiv geladenen Schichten umfasst, die ein biodegradierbares positiv geladenes Polyelektrolyt umfassen und eine Vielzahl von negativ geladenen Schichten, die ein biodegradierbares negativ geladenes Elektrolyt umfassen.

## Revendications

1. Endoprothèse implantable comprenant
- une région métallique en magnésium ou alliage de magnésium biodégradable (100) et
- un revêtement résistant à la corrosion polymère biodégradable (110) sur la région métallique biodégradable (100) qui réduit la vitesse de corrosion de la région métallique biodégradable (100) lors de l'implantation chez un sujet,
dans laquelle ledit revêtement résistant à la corrosion (110) comprend un polymère électriquement conducteur.

2. Endoprothèse implantable selon la revendication 1, dans laquelle la région métallique biodégradable correspond à une endoprothèse implantable entière, ou à un composant d'une endoprothèse implantable.

3. Endoprothèse implantable selon la revendication 1, comprenant une pluralité desdites régions métalliques biodégradables, ou comprenant une pluralité desdits revêtements résistants à la corrosion, ou dans laquelle ledit revêtement résistant à la corrosion est disposé sur seulement une partie de ladite région métallique biodégradable, ou dans laquelle ledit revêtement résistant à la corrosion varie en épaisseur le long d'une surface de ladite endoprothèse implantable, ou dans laquelle ledit revêtement résistant à la corrosion varie en composition le long d'une surface de ladite endoprothèse implantable.

4. Endoprothèse implantable selon la revendication 1, dans laquelle ledit revêtement polymère comprend un additif choisi parmi un agent tampon de pH, un agent chélateur, un agent anticorrosion, un agent facilitant le transport et les associations de ceux-ci.

5. Endoprothèse implantable selon la revendication 1, dans laquelle ledit revêtement résistant à la corrosion comprend un produit de contraste pour l'imagerie, de préférence comprend des particules d'or.

6. Endoprothèse implantable selon la revendication 1, dans laquelle ledit revêtement résistant à la corrosion comprend un agent thérapeutique.

7. Endoprothèse implantable selon la revendication 1, dans laquelle ledit polymère électriquement conducteur est choisi parmi les homopolymères, copolymères et dérivés d'aniline, de pyrrole, de thiophène, de phénylènevinylène, d'anisidine et de thioflavine ainsi que les associations de ceux-ci.

8. Endoprothèse implantable selon la revendication 1, dans laquelle ledit polymère électriquement conducteur est biodégradable.

9. Endoprothèse implantable selon la revendication 1, dans laquelle ledit revêtement résistant à la corrosion comprend des régions dudit polymère électriquement conducteur dans une matrice polymère biodégradable, de préférence dans laquelle ladite matrice polymère biodégradable comprend un polymère biodégradable choisi parmi les polyesters, les polyanhydrides et les polymères biodégradables à base d'acides aminés.

10. Endoprothèse implantable selon la revendication 1, dans laquelle ledit revêtement résistant à la corrosion comprend une espèce dopante chargée.

11. Endoprothèse implantable selon la revendication 1, dans laquelle ledit revêtement résistant à la corrosion comprend une espèce chargée choisie parmi un inhibiteur de corrosion, un agent thérapeutique et les associations de ceux-ci.

12. Endoprothèse implantable selon la revendication 1, dans laquelle ledit revêtement résistant à la corrosion comprend des particules chargées, de préférence dans laquelle lesdites particules chargées sont choisies parmi les particules qui comprennent une espèce choisie parmi les polymères conducteurs, les agents radio-opaques, les polyoxométallates, les agents thérapeutiques, les agents anticorrosion et les associations de ceux-ci.

13. Endoprothèse implantable selon la revendication 1, dans laquelle ledit revêtement résistant à la corrosion comprend
(a) une pluralité de couches positivement chargées comprenant un polyélectrolyte positivement chargé,
(b) une pluralité de couches négativement chargées comprenant un polyélectrolyte négativement chargé ou
(c) une pluralité de couches positivement chargées comprenant un polyélectrolyte positivement chargé et une pluralité de couches négativement chargées comprenant un polyélectrolyte négativement chargé.

14. Endoprothèse implantable selon la revendication 13, dans laquelle ledit revêtement résistant à la corrosion comprend de 10 à 200 couches, ou dans laquelle ledit revêtement résistant à la corrosion comprend en outre une couche qui comprend des particules chargées, de préférence dans laquelle lesdites particules chargées sont choisies parmi les particules qui comprennent une espèce choisie parmi les polymères électriquement conducteurs, les agents radio-opaques, les polyoxométallates, les agents thérapeutiques, les agents anticorrosion, les agents tampons de pH, les agents chélateurs, les agents facilitant le transport et les associations de ceux-ci, ou comprenant un polyélectrolyte qui comprend un agent choisi parmi les agents radio-opaques, les agents thérapeutiques, les agents anticorrosion, les agents tampons de pH, les agents chélateurs, les agents facilitant le transport et les associations de ceux-ci, ou comprenant un polyélectrolyte qui comprend un polymère conducteur, de préférence dans laquelle ledit polymère conducteur est choisi parmi le polypyrrole, la polyaniline et les associations de ceux-ci, ou dans laquelle ledit revêtement résistant à la corrosion varie en porosité avec une variation de pH, ou dans laquelle ledit revêtement résistant à la corrosion diminue en porosité avec une augmentation de pH, ou dans laquelle ledit revêtement résistant à la corrosion comprend une pluralité de couches positivement chargées comprenant un polyélectrolyte positivement chargé biodégradable et une pluralité de couches négativement chargées comprenant un polyélectrolyte négativement chargé biodégradable.
